(19) Europäisches Patentamt
European Patent Office
Office européen des brevets

(11) Publication number : **0 500 236 A1**

# (12) EUROPEAN PATENT APPLICATION

(21) Application number : **92301009.4**

(22) Date of filing : **06.02.92**

(51) Int. Cl.$^5$ : **C07K 7/08,** G01N 33/576, G01N 33/577, C12P 21/08, // C12N15/51

(30) Priority : **08.02.91 JP 104010/91**

(43) Date of publication of application : **26.08.92 Bulletin 92/35**

(84) Designated Contracting States : **BE CH DE DK FR GB IT LI NL**

(71) Applicant : **IMMUNO JAPAN INC.**
**4-28-14-701, Ogikubo Suginami-Ku**
**Tokyo 167 (JP)**

(72) Inventor : **Nakamura, Tetsuo**
**14-701, 4-chome, 28, Ogikubo**
**Suginami-ku, Tokyo 167 (JP)**
Inventor : **Mishiro, Shunji**
**9-301 1-chome, 24 Honkomagone**
**Bunkyo-ku, Tokyo (JP)**

(74) Representative : **Arthur, Bryan Edward**
**Withers & Rogers 4 Dyer's Buildings Holborn**
**London EC1N 2JT (GB)**

## (54) Non-A, non-B hepatitis related nucleic acids, antigens, antibodies and their detection reagents.

(57) Oligopeptide spGORb-epi from non-A, non-B hepatitis (NANB) virus ; detection reagents for antibody associated with NANB hepatitis using the aforementioned oligopeptide or fusion protein of such oligopeptide and conventional proteins ; monoclonal and polyclonal antibodies specific NANB hepatitis obtained by immunization of animals with the aforementioned oligopeptide as antigen ; and detection reagents of NANB hepatitis related antigens using the aforementioned specific antibodies.

**Fig. 1**

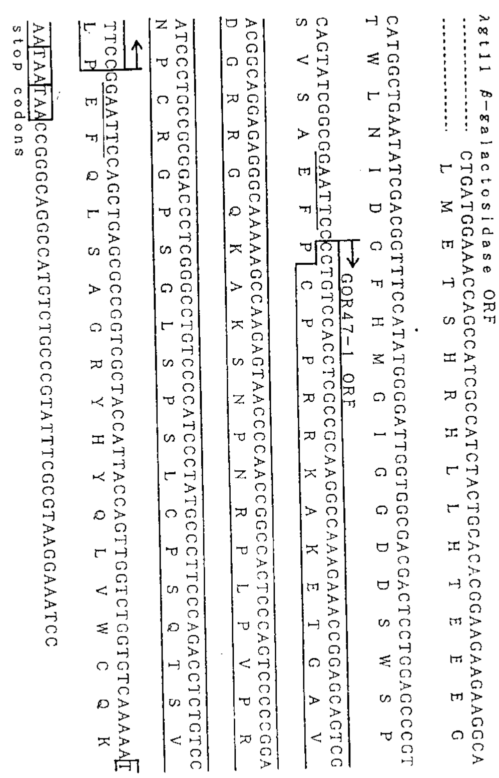



Jouve, 18, rue Saint-Denis, 75001 PARIS

## Reference to Related Applications

The present invention is a continuation-in-part of U.S. Patent Application Serial No. 07/653,090, filed February 8, 1991, and of U.S. Patent Application Serial No. 07/798,226, filed on November 27, 1991, which are relied on and incorporated by reference.

## Introduction to the Invention

The present invention relates to novel antigens, antibodies and non-A, non-B hepatitis diagnostic reagents using those materials.

The causative agent of non-A, non-B hepatitis (hereinafter NANB hepatitis) afflicts human beings with such diseases as acute hepatitis, fulminant hepatitis, chronic hepatitis, liver cirrhosis and hepatocellular carcinoma. It presents itself as the major cause of post transfusion hepatitis in approximately 10% of the blood recipients in Japan. The causative viruses of hepatitis A and hepatitis B have been isolated and these diseases are under medical control. However, the causative agent of NANB hepatitis is a mystery although its presence was assumed over 10 years ago.

In 1988, a research group at Chiron Corporation in the United States announced that they had succeeded in cloning the gene of the causative agent, and introduced an immunoassay kit for detection of viral antibody specific to NANB hepatitis. That diagnostic reagent is now being evaluated at various hospitals, research institutions and blood centers. According to Chiron's research group, the causative agent is a Flavivirus because of its genomic structure, and they have named it hepatitis C virus (HCV). The data reported to date have confirmed a good correlation between HCV antibody detected by their immunoassay kit and the course of the disease of NANB hepatitis. With respect to the HCV antibody, however, there are problems yet to be solved and elucidated: its insufficient specificity and sensitivity (revealed in several reports), cross reaction between the HCV and a large part of auto-immune hepatitis and hepatitis B, and proof that what they call HCV is the real causative agent of NANB hepatitis.

Non-A, non-B hepatitis specific diagnostic assays based on detection systems using the present invention in this and preceding applications (Japanese Patent Application Nos. 028191/90 filed February 9, 1990, 153887/90 filed June 14, 1990, and 335806/90 filed November 30, 1990) are capable of detecting patients not detected by the kit using Chiron's antigen (Ortho HCV Ab ELISA test: Ortho Diagnostic Systems, Tokyo, Japan).

## SUMMARY OF THE INVENTION

An object of the present invention is to provide previously unidentified antigens and antibodies required for detection of NANB hepatitis.

Nucleic acids and proteins disclosed in the present invention have usefulness in the diagnosis of NANB hepatitis and have been provided only by this invention as totally new materials.

The present invention discloses GOR gab DNA having the sequence as shown in list 1; GOR gab protein having the sequence as shown in list 2; GOR47-1 RNA having the sequence as shown in list 3; GOR 47-1 RNAC having the sequence as shown in list 4; GOR47-1 DNA having the sequence as shown in list 5; GOR47-1 DNAC having the sequence as shown in list 6; GOR47-1 Protein having the sequence as shown in list 7; oligopeptide spGOR1 having the sequence as shown in list 8; oligopeptide spGOR2 having the sequence as shown in list 9; oligopeptide spGORa-epi having the sequence as shown in list 10; oligopeptide spGORb-epi having the sequence as shown in list 11; detection assay for nucleotides related to NANB hepatitis using such nucleotides as probe; detection assay for nucleotides related NANB hepatitis using such nucleotides as primer; detection assay for antibodies related to NANB hepatitis using such proteins or peptides as antigen; detection assay for antibodies related to NANB hepatitis using fusion protein with such proteins or peptides as antigen; monoclonal or polyclonal antibodies obtained by immunization of animals with such proteins or peptides; and detection assay for NANB hepatitis related antigen using such monoclonal and polyclonal antibodies. These detection assays can be used as efficient tools for diagnosis of NANB hepatitis.

## BRIEF DESCRIPTION OF THE DRAWINGS

Figure 1 shows the base sequence of recombinant lambda-gtll phage DNA to assemble GOR-47 DNA in EcoRI cleavage. Sequence within the frame is GOR47-1 DNA and those on the left and right sides of the frame are lambda-gtll DNA.

Figure 2 shows reactivity the partial peptide on the ORF of GOR47-1 determined by EIA. Samples with ○ are NANB hepatitis and those with ● are hepatitis B derived; βGal/GOR is fusion protein of β-galactosidase

and GOR47-1; spGOR3 is the peptide extended by 26 residues from the 15th residue of spGORa-epi toward C terminus and having the sequence LPVPRNPCRGPSGLSPSLCPSQTSVL.

Figure 3 shows G1 and G2 used as primers for PCR.

Figure 4 is a histogram showing GORa antibody in samples from normal subjects and chronic hepatitis patients detected by EIA using spGOR2 as antigen. On the ordinate is samples from normal subjects and chronic hepatitis patients detected by EIA using spGOR2 as antigen. On the ordinate is shown frequency. Absorbance 0.4 at $OD_{492}$ is tentatively set as the cut-off value and shown by a dotted line. Positive rate for each group is as follows: (1) Normal subjects-1%; (2) NANB chronic hepatitis patients-76%; (3) Chronic hepatitis B patients-2%; (4) NANB liver cirrhosis-56%; (5) Hepatitis B liver cirrhosis-7%; (6) NANB hepatoma-56%; (7) Hepatitis B hepatoma-0%; (8) Lupoid hepatitis-0%; (9) Primary biliary cirrhosis-0%.

Figure 5 shows change with time of GORa antibody in acute NANB hepatitis patients determined by EIA using spGOR2. ○ : IgG class GORa antibody; ● : IgM class GORa antibody; □ : HCV by Ortho HCV ELISA Ab test kit; black area: Serum ALT (alanine aminotransferase) value.

Figure 6 is distribution pattern of chronic NANB hepatitis patient sera showing positive for GORa antibody. Sera positive for Chiron's HCV antibody are shown by * mark.

Figure 7, a histogram, shows comparison of antibody to GORb epitope detection frequency using spGORb-epi in three groups: 1) normal subjects, 2) type-B liver diseases and 3) NANB hepatitis. On the ordinate is samples from normal subjects and chronic hepatitis patients detected by EIA using spGORb-epi as antigen. On the ordinate is shown frequency. Absorbance 0.4 at $OD_{492}$ is tentatively set as the cut-off value and shown by a dotted line.

Figure 8 shows agarose gel electrophoresis patterns of the material produced by PCR.

## DETAILED DESCRIPTION OF THE INVENTION

One feature of the present invention resides in the following:

Oligopeptide spGORb-epi (list 11) having the following amino acid sequence:

VAKQHVRDGR KDSLDGFV(Y)

18 amino acid residues correspond to amino acid numbers 346-363 of GOR gab protein, while C-terminal (Y) was added for peptide design. Generally (Y) is tyrosine for radio-labelling, though another amino acid(s) can be utilized (e.g. cysteine) for combining with such a protein as alkaline phosphatase.

A, G, C, and U stand for Adenine, Guanine, Cytosine and Uracil respectively in the ribonucleic acids of this invention.

A, G, C, and T stand for Adenine, Guanine, Cytosine and Thymine respectively in the deoxyribonucleic acids of this invention.

C, P, R, K, A, E, T, G, V, D, Q, S, N, L, stand for Cysteine, Proline, Arginine, Lysine, Alanine, Glutamic acid, Threonine, Glycine, Valine, Aspartic acid, Glutamine, Serine, Asparagine and Leucine respectively in the proteins of this invention.

The following procedures were utilized:

### 1) Preparation of animal models for NANB hepatitis

Research started with preparation of animal models for NANB hepatitis. Various animals were injected with sera from donors which were known to have caused post transfusional NANB hepatitis in man. Among those animals, only chimpanzees responded and expressed symptoms similar to human NANB hepatitis. In case of the experimental NANB hepatitis in chimpanzees, some characteristic ultrastructural changes (e.g., convoluted curved membrane, tubular structure, sponge-like structure, and microtubular aggregates) tend to appear in the cytoplasm of liver cells before clinical symptoms, thus making these ultrastructural changes a reliable marker; experimental passage of NANB causative agent was undertaken from a human being to a chimpanzee, then from that chimpanzee to other chimpanzees.

### 2) Material for isolation of NANB hepatitis related gene

Human serum (No. 30017) was injected into a chimpanzee (C37) and the serum from that chimpanzee (in acute phase hepatitis) was injected into 5 chimpanzees (C41, C43, C45, C46 and C47). Among those 5 chimpanzees, plasma were taken from those in the phase positive for the aforementioned ultrastructural changes and pooled for injection into other animals including the chimpanzee CH19. Plasma taken from CH19 (in acute phase of NANB hepatitis) was used as the material for isolation of NANB hepatitis related gene.

3) Extraction of nucleic acids form chimpanzee plasma

About 6 liters of plasma from the chimpanzee CH19 (in acute phase of NANB hepatitis) was centrifuged on the J6 rotor (Beckman) at 3,000 rpm for 30 minutes, and the resulting supernatant was centrifuged on the Ti-15 rotor (Beckman) at 4°C at 30,000 rpm for 5.3 hours. The resulting precipitate was suspended in 120 ml of 50 mM Tris-Cl (pH7.5)/5mM EDTA to obtain virus-rich fraction concentrated by approximately 50 times. After addition to 6 ml of this fraction (equivalent to 300 ml of the original plasma) of 6 ml of SDS/Proteinase K cocktail (400mM NaCl/20mM EDTA/4% SDS/100mM Tris-Cl buffer (pH 8.0)/Proteinase K 2 mg/ml) and overnight incubation at 37°C, nucleic acids were extracted with phenol and precipitated by ethanol.

4) cDNA synthesis

Using 1/4 in volume (equivalent to 75 ml of the original plasma) of the nucleic acids obtained under 3) above, cDNA was synthesized. After incubation of the template RNA at 65°C for 3 minutes, primary cDNA strand was synthesized in two tubes, one tube with oligo-$dT_{12}$, and the other with randam-hexanucleotide as primer. This series of cDNA synthesis, including synthesis of the secondary cDNA strand and blunting of double-stranded DNA termini, was conducted according to Gubler's method using a cDNA kit (Amersham, U.K.)

5) Preparation of cDNA library

After protecting possible EcoRI cleavage sites in the double-stranded cDNA prepared in 4) above by EcoRI methylase, providing both ends with EcoRI linker, it was integrated into lambda-gtll DNA at the EcoRI site and assembled with phage protein to make recombinant phage. Series of this reaction was processed using the lambda-gtll cloning kit (Amersham, U.K.). Library size of cDNA primed by oligo $dT_{12}$ and that of cDNA primed by randam-hexanucleotide were 1.0 x $10^6$ PFU and 4.3 x $10^6$ PFU respectively.

6) Screening of cDNA library

cDNA libraries prepared under 5) were searched for a NANB hepatitis related cDNA clone by immunoscreening using two antibodies. After infecting E.coli Y1090 with the above recombinant phage, and dispensing it on LB-Agar plates for incubation at 43°C for 3 hours, a filter impregnated with IPTG was placed on it and incubated at 37°C for 3 hours. The filter was then removed and washed with the buffer and primary antibody was added to it. A mixture of plasma of a chimpanzee infected with NANB hepatitis, a human plasma known to have caused NANB hepatitis by needle stick accident, and a plasma of chronic NANB hepatitis patient were used as the primary antibody, and incubated at 4°C overnight. After washing with the buffer and addition of the secondary antibody (peroxidase labeled antihuman IgG), it was incubated at room temperature for 30 minutes. It was then washed with the buffer and added with the mixture of DAB (3,3′-Diaminobenzidine tetrahydrochloride; Sigma, USA), Ni, Co, $H_2O_2$ for color development. If there were tractions of NANB related genes in the cDNA libraries, and if they fused with the open reading frame (ORF) of β-galactosidase of lambda-gtll phage DNA in-frame, there should be fusion protein expressed by E.coli infected with the phage and, upon its recognition by NANB related antibody, it would bind to the antibody to give a positive signal. Inventors' experiment confirmed that point.

Among several clones giving positive signals in the screening test, there was a clone named GOR47-1 described hereafter.

7) Determination of the nucleotide sequence of NANB hepatitis related cDNA clone (GOR47-1)

The nucleotide sequence of GOR47-1 obtained under 6) above was determined by Sanger's method by purifying it, cleaving its DNA by EcoRI to take out cDNA, and subcloning it to EcoRI site of Phagescript (trade name of Stratagene, USA). By means of primer derived from DNA phage, the nucleotide sequence at the insertion site of GOR47-1 was simultaneously determined in the same method. As a result, it was confirmed that GOR47-1 DNA was inserted into lambda-gtll DNA by the sequence and direction shown in Figure 1. Figure 1 shows the nucleotide sequence of GOR47-1 with the junctional sequence of lambda-gtll DNA. Sequence within the box is for GOR47-1 and those on the left and right sides of the box are for the lambda phage.

8) Determination of the primary structure of the protein coded for by NANB hepatitis related cDNA clone (GOR47-1)

From the nucleotide sequence determined under 7) above, ORF of GOR47-1 linked with ORF of β-galactosidase of lambda-gtll in frame was determined as per Figure 1. Lambda-gtll phage DNA used for preparation of cDNA libraries from which GOR47-1 was derived is a so called expression vector and expresses fusion protein of β-galactosidase and cDNA derived protein by assembling cDNA in operon of lacZ gene of lambda-gtll. Out of 6 possible reading frames, only one frame can fuse in frame with ORF of β-galactosidase of lambda-gtll as shown in Figure 1. There is no stop codon in this ORF encoding 55 amino acids. Under this invention, β-galactosidase can be replaced by such expression proteins as alkaline phosphatase and superoxide dismutase.

9) Search for cDNA clone overlapping in sequence with NANB hepatitis related cDNA clone (GOR47-1)

From GOR47-1 phage, GOR47-1 DNA and GOR47-1 DNAC. were cut out as insert DNA, and from recombinant phagescript obtained, GOR47-1 RNA and GOR47-1 RNAC were prepared by $T_3$ and $T_7$ promoters. They were all labeled with radioisotope. When they were used as probes to screen the cDNA libraries prepared under 5) above, cDNA clone (GOR gab DNA), while overlapping with GOR47-1, had a longer sequence. The base sequence of GOR gab DNA was determined in the same way as described above.

GOR gab DNA has the nucleotide sequence shown in list 1. GOR 47-1 DNA corresponds to nucleic acid numbers 1703-1868 of GOR gab DNA.

10) GOR gab protein encoded by NANB hepatitis related gene GOR gab DNA

GOR gab protein which contains 661 amino acids was obtained by search for a sequence overlapping with GOR 47-1 ORF from 6 possible ORF which were coded in GOR gab DNA.

List 2 shows the amino acid sequence of GOR gab protein. The ORF coded by GOR 47-1 corresponds to amino acid numbers 569-623 in the sequence of GOR gab protein.

11) Determination of NANB hepatitis related epitope (GORa) included in GOR gab Protein

The following experiments were conducted using fusion proteins and synthetic peptides in order to determine NANB hepatitis related epitope.

(11-1) Purification of fusion protein made by NANB hepatitis related cDNA clone GOR47-1 and lambda-gtll and β-galactosidase

*E. coli* Y1089 was lysogenically infected with the NANB hepatitis related gene (GOR47-1) obtained under 6) above to make lysogen. (Lysogen was prepared according to the method described in "Constructing and Screening cDNA Libraries in lambda-gtll", Thanh V. Huyuh et al., DNA Cloning Vol. 1, a practical approach, ed. by D.M. Clover, pages 949-78, IRL Press, Oxford, 1985.) After culturing and inducing expression of protein by IPTG (isopropyl-β-D-thiogalactoside), and destruction of the lysogen by means of freezing-thawing and sonication, lysate was obtained and subjected to an affinity chromatography column (Sepharose 4B Immobilized IgG fraction Rabbit anti-β-galactosidase; Cappel, USA) to obtain the fusion protein.

(11-2) Preparation of synthetic peptides originated in GOR47-1 ORF

The following three peptides were synthesized by Merrifield's solid phase method. Numbers indicate positions in the sequence of GOR gab protein.

spGOR1 (list 8) Nos. 569-597

CPPRRKAKET GAVDGRRGQK AKSNPNRPL 29

spGOR2 (list 9) Nos. 583-609

GRRGQKAKSN PNRPLPVPRN PCRGPSG 27

spGOR3 Nos.597-622

LPVPRNPCRG PSGLSPSLCP SQTSVL 26

(11-3) Epitope mapping by EIA (Enzyme Immuno Assay) and spGORa-epi

As described in the examples, antibody against fusion protein and synthetic peptides prepared in (11-1) and (11-2) above were detected by the EIA method. As Figure 2 shows, fusion protein (β-Gal/GOR), spGOR1 and spGOR2 had identical antigenicity, but spGOR3 did not.

According to this result, epitope was identified as the following sequence of oligopeptide spGORa-epi (list 10) shared with fusion protein and two synthetic peptides.

GRRGQKAKSNPNRPL

Oligopeptide spGORa-epi was obtained by synthesis.

12) Another epitope on GOR gab Protein, spGORb-epi

The two most hydrophilic regions were found in GOR gab protein provided in 10) above. One of them was spGORa-epi of (11-3). Another region was revealed to have NANB hepatitis related epitope from the result of experiments concerning reactivity against antibody, when using synthetic peptide of the region, as shown in the examples.

The amino acid sequence of the synthetic oligopeptide, spGORb-epi, was as follows;

VAKQHVRDGR KDSLDGFV(Y)

18 amino acid residues correspond to numbers 346-363 of GOR gab protein, while C-terminal (Y) was added for peptide design. Generally (Y) is tyrosine for radiolabelling, though another amino acid(s) can be utilized (e.g. cysteine) for combining with such a protein as alkaline phosphatase.

13) Preparation of antibody specific to NANB hepatitis related antigen

Monoclonal and polyclonal antibodies were obtained by immunizing mice, guinea pigs, goats, horses, etc. with fusion proteins and synthetic peptides, GOR gab Protein, (GOR 47-1 Protein, spGOR1, spGOR2, spGORa-epi and spGORb-epi bearing the NANB hepatitis related epitope which were determined under 11) and 12) above.

14) Preparation of antigen assay by specific antibody to the NANB hepatitis related antigen

Antibodies prepared under 13) above were labeled with FITC (fluorescein isothiocyanate) to make a probe for staining sample sections, such as liver tissue of patients infected with NANB hepatitis, to locate or examine locality of antigen specific to NANB hepatitis in tissue. An assay system was also developed by labelling specific antibodies with peroxidase or biotin to detect NANB hepatitis related antigen in patient's sera or plasma. This assay employs the "sandwich method" in which microplates or beads are coated with specific antibodies, and samples and labeled antibodies are applied in sequence for reaction.

15) Detection of NANB hepatitis related gene by Polymerase Chain Reaction (PCR)

According to the method described under 3) above, viral fractions were obtained by centrifugation of sample sera or plasma and subjected to PCR after treatment with SDS/Proteinase K cocktail and extraction of nucleic acid with phenol. Primer used for PCR was oligonucleotides G1 and G2, each consisting of 20 nucleotides as shown in Figure 3 and located at both ends of NANB hepatitis related cDNA GOR47-1. G1 corresponds to 20 bases of 5′ terminus of GOR47-1 DNA and is closest to 5′ side of the sense strand, while G2 corresponds to 20 bases of 5′ terminus of GOR47-1 DNAC and is closest to 5′ side of anti-sense strand. As a result, the presence of PCR product of expected length (same 166 bases as GOR 47-1) was confirmed in both chimpanzee and human plasma which was suspected to be NANB hepatitis.

Examples

1) GOR gab DNA.

The nucleotide sequence of NANB hepatitis related cDNA (GOR47-1) was determined in the following way.

GOR47-1 phage obtained in the above-mentioned 6) was purified, its DNA cleaved by EcoRI to obtain cDNA, that cDNA subcloned to EcoRI site of Phagescript (Stratagene, USA) and its nucleotide sequence determined by Sanger's method. Nucleotide sequence of the linking part of the insert arm of GOR47-1 phage DNA was similarly determined by the primer derived from the phage DNA. Those sequences revealed that GOR47-1 DNA was inserted in the lambda-gtll DNA with the nucleotide sequence and direction shown in Figure 1, and insert DNA, that is GOR47-1 DNA, was cut out from DNA47-1 phage DNA. Figure 1 shows the nucleotide sequence of GOR47-1 together with its linking part with lambda-gtll DNA. The sequence within the box is that of GOR47-1 and sequences on the right and lett side of the box are those of lambda-gtll DNA.

GOR47-1 DNA has the nucleotide sequence shown in list 5.

In the similar way, GOR47-1 DNAC (having DNA complementary to strain GOR47-1 DNA) was cut out from GOR47-1 phage DNA. GOR47-1 DNAC has the nucleotide sequence shown in list 6.

In the next step, GOR47-1 RNA and GOR47-1 RNAC (which have the nucleotide sequences shown in list 3 and list 4) were prepared from recombinant phage script of GOR47-1 DNAC using $T_3$ and $T_7$ promoters, they were labeled with radioisotope and used as probe to search the cDNA libraries prepared in 5) above, and GOR gab DNA, clone of cDNA, overlapping with but having longer sequence than GOR47-1, was obtained. The nucleotide sequence of GOR gab DNA (list 1) was determined in the same way as above.

It is well known in the art that one or more nucleotides in a DNA sequence can be replaced by other nucleotides in order to produce the same protein. The present invention also concerns such nucleotide substitutions which yield a DNA sequence which codes for GOR gab Protein (the amino acid sequence of which is described above).

2) GOR gab Protein.

From GOR gab DNA, GOR gab Protein coded by it was obtained. Lambda-gtll phage DNA used for preparation of cDNA libraries from which GOR gab DNA was derived is a so called "expression" vector and expresses fusion protein of β-galactosidase and cDNA derived protein by assembling cDNA in operon of lacZ gene of lambda-gtll. Among possible reading frames of GOR gab DNA, only one reading frame fuses in frame with open reading frame (ORF) of β-galactosidase of lambda-gtll.

Amino acids sequence of GOR gab Protein are shown in list 2. It is well known in the art that one or more amino acids in an amino acid sequence can be replaced by equivalent other amino acids, as demonstrated by U.S. Patent No. 4,737,487 which is incorporated by reference, in order to produce an analog of the amino acid sequence. Any analogs of GOR gab Protein of the present invention involving amino acid deletions, amino acid replacements, such as replacements by other amino acids, or by isosteres (modified amino acids that bear close structural and spatial similarity to protein amino acids), amino acid additions, or isosteres additions can be utilized, so long as the sequences elicit antibodies recognizing NANB antigens.

In this invention, β-galactosidase can be substituted by such expression proteins as alkaline phosphatase and superoxide dismutase.

3) NANB hepatitis antigen related epitope by synthetic peptide based on the amino acid sequence coded for by GOR gab DNA.

Synthetic peptides of various lengths and for various regions based on the ORF of GOR gab DNA were prepared and examined for reactivity with NANB hepatitis related antibody to determined the location of NANB hepatitis epitope. Polystyrene microplates coated with synthetic peptide prepared by Merrifield's solid phase method as solid phase, sample sera as primary antibody, and peroxidase labeled anti-human IgG or IgM antibodies as secondary antibody for color reaction were used.

As a result, spGORa-epi and spGORb-epi were determined as NANB hepatitis related epitopes. SpGORa-epi has the amino acid sequence shown in list 10 and spGORb-epi shown in list 11.

4) NANB hepatitis antibody detection system using proteins and peptides described above as antigen.

An example of experiments of NANB hepatitis antibody detection system (Enzyme Immuno Assay) using GOR gab protein and peptides is shown below.

50 μl each of GOR gab Protein or peptides having 10 or more amino acids residues dissolved in Tris-HCl buffer (10mM, pH 7.5) and adjusted to 5 μg/ml concentration was dispensed in each well on Costar vinyl plates (Toyobo, Japan) and incubated overnight at room temperature (coating of peptide). After washing the wells (0.1% Tween 20, 150mM NaCl; all washing procedures hereafter in this experiment, unless otherwise stated, were with this solution), 100 μl each of the blocking solution (0.1% Tween 20, 150mM NaCl, 30% fetal calf serum) was dispensed in the wells for overnight incubation at 4°C on a vibration free. After washing the wells, 50μl each of sample plasma, or serum preliminary diluted 30 times with the above-mentioned blocking solution, was dispensed in the wells for incubation at room temperature for 30 minutes on a vibrator. After washing the wells, 50μl each of peroxidase labeled anti-human IgG or IgM mouse monoclonal antibody solution was dispensed in the wells for incubation at room temperature for 30 minutes on a vibrator, then peroxidase substrate solution was added for color development and absorbance measurement at wavelength 492nm on a spectrophotometer.

(1) When samples from 100 chronic liver disease patients (mixture of hepatitis B and NANB hepatitis) were assayed by the EIA method, frequency by the assay using the protein or peptides under this invention turned out to be high for NANB chronic hepatitis, liver cirrhosis and hepatocellular carcinoma, while low for chronic liver disease, liver cirrhosis and hepatocellular carcinoma caused by hepatitis B virus, lupoid hepatitis of which autoimmune disorder was suspected, and primary biliary cirrhosis. The assay also showed low frequency for samples from normal subjects, thus proving the efficiency of the protein and peptides under the present invention for use in detection system of NANB hepatitis related antibody.

(2) As Figure 4 shows, GORa antibody frequency detected by EIA using spGOR2 was as high as 76%, 56% and 56% for NANB related chronic hepatitis, liver cirrhosis and hepatoma respectively, while it was as low as 2%, 7% and 0% for hepatitis B related chronic hepatitis, liver cirrhosis and hepatoma respectively. It was 0% and 0% for lupoid hepatitis and primary biliary cirrhosis which were supposed to be closely associated with autoimmune mechanism, or was only 2 (1%) out of 200 for normal subjects. This proved a close relation of GORa antibody with NANB hepatitis.

(3) Change of GORa antibody in an acute NANB hepatitis patient.

GORa antibody positive frequency turned out very high for chronic hepatitis, liver cirrhosis and hepatoma as shown above, and the antibody appeared in very early period for acute hepatitis. A typical example of an outbreak of acute hepatitis is shown in figure 5 (needle stick accident in which a nurse stuck herself with an injection needle while drawing blood from a patient with NANB chronic hepatitis).

In this case, both IgM and IgG classes of GORa antibodies were detected on the 49th day after the needle stick accident while HCV antibody was detected (Ortho HCV Ab ELISA test; Ortho Diagnostic Systems, Japan) as late as on the 131st day after the accident. This has proven that GORa antibody is effective in early detection of infection.

GORa antibody could be derived from GOR 47-1 protein, spGOR1, spGOR2, and spGORa-epi.

5) Detection of NANB hepatitis related antibody by the fusion protein containing NANB epitope spGORa-epi as antigen

While the fusion protein of GOR 47-1 and lambda-gtll-β-galactosidase was used, β-galactosidase can be substituted by such expression proteins as alkaline phosphatase and superoxide dismutase.

The fusion protein obtained above was electrophoresed by SDS-PAGE and its pattern was transferred onto nitrocellulose membrane by the Western blotting technique. Sample plasma was applied onto this membrane and after incubation anti-human immunoglobulin labeled with peroxidase was added for detection of the antibody through color reaction.

The Western blotting method was used for detection system as follows: The nitrocellulose membrane with the electrophoresed pattern transferred onto it was rinsed for 10 minutes, dried naturally, shredded into strips 3.5 mm wide, and those strips were immersed in TBS (50mM Tris-Cl buffer, pH 7.4, 150 mM NaCl) containing 2% skim milk at room temperature for 1 hour or at 4°C overnight (blocking), then washed with TBST (TBS containing 0.05% Tween 20). After immersion at room temperature for 1 hour or at 4°C overnight in the primary antibody diluted 30 times with 3% BSA (primary antigen-antibody reaction), those strips were washed with TBST, immersed at room temperature for 30 minutes in biotinylated anti-human IgG or anti-human IgM (Vectastain ABC kit; Vector Laboratories Inc., USA) diluted with TBS (secondary antigen-antibody reaction), washed with TBST, then immersed in peroxidase labeled mixture of biotin and avidin (Vectastain ABC kit; Vector Laboratories Inc., USA) at room temperature for 30 minutes (biotin-avidin reaction), then washed with TBST followed finally by addition of coloring reagent to examine the presence of reactive substances. The coloring reagent was prepared by adding 25 mg of DAB (3,3′-Diaminobenzidine tetrahydrochioride; Sigma, USA) to 50 ml of 50 μM $NaPO_4$ pH7.4 and, after dissolution of DAB, 1 ml of 5% $CoCl_3$-$6H_2O$, 5% $(NH_4)_2Ni(SO_4)_2 \cdot 6H_2O$, then, 50 μl of 30% $H_2O_2$. When antibody was positive, a band of about 120 K Dalton was stained dark blue.

(1) Detectability of NANB hepatitis related antibody (GORa antibody) in patients with chronic liver diseases.

Sera of 100 patients with chronic liver diseases (40 cases of hepatitis B and 60 cases of non-B hepatitis) were assayed by the Western blot analysis. Results are shown in Tables 1 and 2, and Figure 6. As shown in Tables 1 and 2, among 100 cases, GORa antibody was positive for 52 cases in total and its rate was much higher for the sera of non-B hepatitis cases (80%) than for the sera of hepatitis B cases (10%). Especially in chronic non-B hepatitis cases, GORa antibody was positive for 27 cases out of 30 cases (90%). When GORa antibody was compared with Chiron's HCV antibody (Ortho HCV Ab ELISA test; Ortho Diagnostic Systems, Tokyo, Japan), the former showed lower positive rate for the sera of hepatitis B cases (10%, 13%) and conversely higher positive rate for sera of NANB hepatitis cases (80%, 68%) than the latter. Furthermore, as shown in Figure 6, there were 14 NANB hepatitis cases which GORa antibody detected but HCV antibody failed to detect, while there were only 6 converse cases.

(2) GORa antibody in sera from donors having abnormal liver function.

Frequency of GORa antibody and HCV antibody for sera of randomly selected 42 donors (ALT over 80, hepatitis B negative) were, as shown in Table 3, 20% and 17% respectively. Sera were tested in the method described in 1) immediately above. In five cases, both GORa antibody and HCV antibody were positive. ALT (alanine aminotransferase) level in serum is one of the markers which indicate liver function and is high (abnormal) when the host is infected with viral hepatitis, but it is also high when liver is malfunctioning due to some other causes such as alcohol, drugs, obesity, etc. The group consisting of 42 donors was consciously healthy but was unconsciously high in ALT level and differed from the groups in (1) above in that the cause of the high ALT level was not checked in this group while that of the groups in (1) was checked (chronic hepatitis, liver cirrhosis, or hepatoma). The difference in frequency of GORa antibody between this group and those in (1) may be attributed to this fact.

(3) Comparison of EIA for the detection of GORa antibody using synthetic peptide and Western blot analysis using protein fused with β-galactosidase.

100 sera from patients with chronic type-B or type-NANB liver diseases were tested by EIA for GORa antibody and most of them turned out to be positive by both EIA and Western blot analysis, but 11 samples were positive by EIA but negative or weakly positive by Western blot analysis, suggesting that the former detects GORa antibody at higher sensitivity than the latter (Table 5).

6) <u>The antibody detection system using spGORb-epi which contains NANB hepatitis related epitope of the invention, GORb, as antigen was prepared in the same method as in example 1).</u>

The results shows the efficiency of spGORb-epi for detection of NANB hepatitis as follows; GORb antibody was detected in 8 out of 290 cases (3%) in normal subjects, 1 out of 36 cases (3%) in type-B liver disease and 17 out of 54 cases (31%) in NANB hepatitis as shown in Fig 7 (cutoff value was $OD_{492}$ = 0.4.

7) <u>Preparation of monoclonal and polyclonal antibodies.</u>

Specific monoclonal and polyclonal antibodies were obtained by immunizing such animals as mice, guinea pigs, rabbits, goats and horses with GOR gab Protein and the synthetic peptides bearing NANB hepatitis antigenic epitope described in the above example.

8) Antigen detection assay using antibody specific to NANB hepatitis related antigen.

By staining tissue sections of liver, etc. from NANB hepatitis patients with the aforementioned specific antibody labeled with FITC as probe, the presence and locality of NANB hepatitis specific antigen in tissue was examined. By labeling the specific antibody with peroxidase or biotin, assay system for detection of NANB related antigen in patient serum or plasma was developed. The system was the sandwich method in which polystyrene microplates or beads coated with NANB hepatitis specific antibody were used as solid phase and after addition of samples for reaction with the solid phase, labeled specific antibody was added for the second reaction.

Experiments of those assays confirmed the effectiveness of monoclonal and polyclonal antibodies under the present invention for detection of NANB hepatitis related antigen.

9) Detection of NANB hepatitis related gene by Polymerase Chain Reaction (PCR)

According to the method described under 3) above, viral fractions were obtained by centrifugation of sample sera or plasma and subjected to PCR after treatment with SDS/Proteinase K cocktail and extraction of nucleic acid with phenol. Primer used for PCR was oligonucleotide G1 and G2, each consisting of 20 nucleotides as shown in Figure 3 and located at both ends of NANB hepatitis related cDNA GOR47-1. G1 corresponds to 20 bases of 5′ terminus of GOR47-1 DNA and is closest to 5′ side of the sense strand, while G2 corresponds to 20 bases of 5′ terminus of GOR47-1 DNAC and is closest to 5′ side of anti-sense strand. After addition of primer G2 (antisense) to the above mentioned nucleic acid fractions as the primary reaction in PCR, 4 nucleotides and transcriptase were added for reaction and synthesis of primary cDNA. Primer G1 was then added and the standard PCR reaction was followed for 36 cycles using Taq polymerase and the automatic temperature control unit (Cetus Corp., U.S.). The product (DNA) thus produced was separated by agarose gel electrophoresis and the presence of an expected length (same 166 bases as GOR47-1) of DNA fragment (identified by * in Figure 8) was confirmed. Chimpanzee (CH19) plasma from which NANB hepatitis related cDNA clone GOR47-1 was derived contained PCR product of 166 bases (Figure 8, Lane 1). A similar band was separated from the plasma of chimpanzee (CH413) having known infectious unit of $10^7$ CIU/ml (chimpanzee infectious units/ml) (Figure 8, Lane 2). Human plasma (T.S) known to have $10^6$ CIU/ml also showed the similar length of band but its amount was about 1/10 of that of the aforementioned chimpanzee having $10^7$ CIU/ml. Patterns on the extreme left of the figure are those of molecular markers.

10) Seroconversion of GORa antibody in acute NANB hepatitis patient

Table 4 contains data from a nurse who had been accidentally exposed to a needle stick contaminated with blood from a non-A, non-B hepatitis patient resulting in an acute hepatitis. As the table shows, there was no GORa antibody before infection and it was detected only after infection. At the initial stage of infection, GORa antibody of the IgM class was also positive. Emergence of HCV antibody, on the other hand, was very late. At the 8th week after infection, GORa antibody was already positive, while Chiron's HCV antibody was negative (Ortho HCV Ab ELISA test; Ortho Diagnostic Systems, Tokyo, Japan) until the 17th week after infection; this proves that, at least for this case, serological diagnosis of NANB hepatitis infection at an early stage is possible only with GORa antibody. Expression of IgM class GORa antibody in the acute phase presents another important significance. It is not easy to discern between an acute exacerbation of chronic hepatitis and acute hepatitis. They can, however, be distinguished by testing for GORa antibody IgM class and accurate diagnosis becomes possible.

The antigens and antibodies of the invention provide diagnostic reagents capable of quicker and wider range of diagnosis of NANB hepatitis than any other conventional reagent, and can be used at blood centers, blood derivatives manufacturers, transfusion departments of hospitals and many other places for elimination of blood carrying NANB hepatitis agent from transfusion blood and blood derivatives. Screening tests by the diagnostic reagents using the antigen and antibodies under this invention will provide optimum means for prevention of post transfusion hepatitis which has long been a grave concern.

The nucleic acids and protein under this invention are manufactured not only as important reagents for serological and virological study of NANB hepatitis causative agent and for pathological study of the disease caused by it, but also as indispensable materials for manufacture of the aforementioned antigens and antibodies.

U.S. Patent Application Serial No. 07/540,604, filed on June 19, 1990, is hereby incorporated by reference for its teachings that the materials of the present invention may be utilized in detection kits and as vaccines.

Further variations and modifications of the invention will become apparent to those skilled in the art from the foregoing and are intended to be encompassed by the claims appended hereto.

Japanese Priority Application 104010/91, filed on february 8, 1991, is relied on and incorporated by reference.

Table 1: GORa antibody detected in patients with chronic liver disease

| No. | Patient | | Diagnosis | GORa Antibody | * HCV Antibody |
|---|---|---|---|---|---|
| 1 | A.O. | (M) | Chronic hepatitis/B | - | - |
| 2 | M.O. | (M) | " | - | - |
| 3 | S.F. | (M) | " | - | - |
| 4 | Y.I. | (M) | " | + | + |
| 5 | H.N. | (M) | " | - | - |
| 6 | T.O. | (M) | " | - | - |
| 7 | S.Y. | (F) | " | - | - |
| 8 | K.G. | (M) | " | - | - |
| 9 | A.I. | (M) | " | - | - |
| 10 | K.N. | (M) | " | - | - |
| 11 | H.K. | (M) | " | - | - |
| 12 | N.T. | (M) | " | - | - |
| 13 | Y.T. | (M) | " | - | - |
| 14 | T.K. | (M) | " | - | - |
| 15 | K.A. | (M) | " | - | - |
| 16 | K.S. | (M) | " | + | + |
| 17 | T.N. | (F) | " | - | - |
| 18 | H.T. | (M) | " | - | - |
| 19 | K.G. | (F) | " | - | - |
| 20 | T.T. | (M) | " | - | - |
| 21 | T.S. | (M) | " | - | - |
| 22 | H.A. | (M) | " | - | - |
| 23 | S.T. | (M) | " | - | - |
| 24 | S.O. | (M) | " | - | - |
| 25 | M.W. | (M) | " | - | - |
| 26 | T.S. | (M) | " | - | - |
| 27 | T.S. | (M) | " | + | - |
| 28 | F.N. | (M) | Chronic hepatitis/Non-B | + | + |
| 29 | G.O. | (M) | " | + | + |
| 30 | M.O. | (M) | " | + | + |
| 31 | R.F. | (M) | " | + | + |
| 32 | S.K. | (F) | " | + | - |
| 33 | Y.M. | (M) | " | + | + |
| 34 | T.Y. | (F) | " | - | + |
| 35 | T.Y. | (M) | " | + | + |
| 36 | Y.K. | (M) | " | - | - |
| 37 | K.I. | (M) | " | + | + |
| 38 | K.O. | (F) | " | + | + |
| 39 | H.S. | (M) | " | + | + |
| 40 | A.H. | (F) | " | + | + |
| 41 | K.I. | (M) | " | + | + |
| 42 | K.G. | (M) | " | - | - |
| 43 | Y.K. | (M) | " | + | + |
| 44 | M.S. | (M) | " | + | + |
| 45 | C.A. | (F) | " | + | - |
| 46 | M.Y. | (F) | " | + | + |
| 47 | K.I. | (F) | " | + | + |
| 48 | R.N. | (M) | " | + | - |
| 49 | F.F. | (F) | " | + | - |
| 50 | T.M. | (M) | " | + | + |

| | | | | | |
|---|---|---|---|---|---|
| 51 | H.K. | (F) | " | + | + |
| 52 | H.S. | (F) | " | + | + |
| 53 | Y.K. | (M) | " | - | + |
| 54 | T.I. | (F) | " | + | + |
| 55 | S. ?. | (F) | Chronic hepatitis/Non-B | + | + |
| 56 | H.M. | (M) | " | + | - |
| 57 | S.A. | (F) | " | + | - |
| 58 | Y.M. | (M) | Liver Cirrhosis/B | - | + |
| 59 | N.Y. | (M) | " | - | - |
| 60 | H.I. | (M) | " | - | - |
| 61 | S.K. | (M) | " | - | - |
| 62 | M.K. | (M) | " | - | - |
| 63 | H.F. | (F) | " | - | - |
| 64 | S.A. | (M) | " | - | - |
| 65 | Y.F. | (M) | " | - | - |
| 66 | T.F. | (F) | " | - | - |
| 67 | K.K. | (F) | " | - | - |
| 68 | T.K. | (M) | " | + | + |
| 69 | M.N. | (M) | Liver Cirrhosis/Non-B | - | + |
| 70 | K.O. | (F) | " | + | + |
| 71 | K.M. | (M) | " | + | + |
| 72 | F.K. | (F) | " | + | + |
| 73 | H.S. | (F) | " | + | + |
| 74 | H.O. | (F) | " | + | + |
| 75 | T.I. | (M) | " | - | + |
| 76 | Y.A. | (F) | " | + | - |
| 77 | T.K. | (M) | " | + | - |
| 78 | S.F. | (M) | " | - | + |
| 79 | Y.I. | (M) | " | + | + |
| 80 | H.H. | (F) | " | + | - |
| 81 | R.S. | (M) | " | + | + |
| 82 | ?.N. | (M) | " | + | + |
| 83 | K.K. | (M) | " | - | + |
| 84 | K.Y. | (M) | Liver Cirrhosis/Non-B | + | - |
| 85 | K.T. | (F) | " | + | + |
| 86 | G.N. | (M) | " | + | + |
| 87 | T.S. | (M) | Hepatoma/B | - | - |
| 88 | N.T. | (M) | " | - | - |
| 89 | T.Y. | (M) | Hepatoma/Non-B | - | - |
| 90 | M.I. | (F) | " | + | + |
| 91 | R.A. | (M) | " | - | + |
| 92 | J.S. | (M) | " | - | - |
| 93 | K.S. | (M) | " | + | - |
| 94 | S.K. | (M) | " | + | - |
| 95 | T.T. | (M) | " | - | - |
| 96 | S.A. | (M) | " | + | + |
| 97 | H.A. | (M) | " | - | - |
| 98 | U.K. | (F) | Hepatoma/B | + | + |
| 99 | ?.O. | (M) | " | + | + |

| | | | | |
|---|---|---|---|---|
| 100 | H.O. (M) | " | + | + |

Remarks:
1) "B" and "Non-B" in the "Diagnosis" column denote patients clearly associated with hepatitis B virus and those not associated with the virus respectively.
2) * column shows result of assays by Ortho HCV Ab ELISA test kit for comparison purpose.
3) (M) and (F) in "Patient" column designate male and female respectively.

Table 2: Frequency of NANB GOR47-1 antibody in patients with liver diseases

| Disease Group | Number of Patient | Frequency of GORa Antibody Number (Rate) | * HCV Antibody |
|---|---|---|---|
| Chronic hepatitis/B | 27 | 3 (11%) | 2 ( 7%) |
| Chronic hepatitis/Non-B | 30 | 27 (90%) | 21 (70%) |
| Liver cirrhosis/B | 11 | 1 ( 9%) | 3 (27%) |
| Liver cirrhosis/Non-B | 18 | 14 (78%) | 14 (78%) |
| Hepatoma/ B | 2 | 0 ( 0%) | 0 ( 0%) |
| Hepatoma/Non-B | 12 | 7 (58%) | 6 (50%) |
| B Total | 40 | 4 (10%) | 5 (13%) |
| Non-B Total | 60 | 48 (80%) | 41 (68%) |
| Grand Total | 100 | 52 (52%) | 46 (46%) |

Remarks:
* column shows for comparison number (rate) of samples tested positive for HCV in Table 1.

Table 3: GORa antibody detected in donors with malfunctioning liver.

| Donor No. | GORa Antibody | * HCV Antibody | Donor No. | GORa Antibody | * HCV Antibody |
|---|---|---|---|---|---|
| 1 | - | - | 22 | - | - |
| 2 | - | - | 23 | + | + |
| 3 | - | - | 24 | + | - |
| 4 | - | - | 25 | - | + |
| 5 | - | - | 26 | - | - |
| 6 | - | - | 27 | - | - |
| 7 | - | - | 28 | - | - |
| 8 | - | - | 29 | - | - |
| 9 | - | - | 30 | + | + |
| 10 | - | - | 31 | - | - |
| 11 | - | - | 32 | + | + |
| 12 | - | - | 33 | - | - |
| 13 | - | - | 34 | - | - |
| 14 | - | - | 35 | - | - |
| 15 | - | + | 36 | + | - |
| 16 | - | - | 37 | - | - |
| 17 | - | - | 38 | + | + |
| 18 | - | - | 39 | - | - |
| 19 | - | - | 40 | - | - |
| 20 | - | - | 41 | N.T. | - |
| 21 | + | + | 42 | + | - |

Remarks:

1) For comparison purpose, * column shows the result of samples tested for Chiron's HCV antibody.

2) All samples were tested negative for hepatitis B virus antigen. As demonstrated in Table 2, 90% of Non-B chronic hepatitis samples were tested positive for GORa antibody, while only 20% of those "consciously healthy" population were positive for the antibody since such liver malfunction derives not only from the virus but from obesity and alcohol.

3) "N.T." means "not tested".

Table 4: Seroconversion of GORa in an acute non-A, non-B hepatitis patient

| Time (Week) | ALT | GORa Antibody | | * (HCV Antibody) |
|---|---|---|---|---|
| | | IgM | IgG | |
| 0 | 13 | - | - | - |
| 4 | 32 | N.T. | N.T. | - |
| 7 | 889 | N.T. | N.T. | - |
| 8 | 381 | + | + | - |
| 11 | 5 | + | ± | - |
| 14 | 20 | N.T. | N.T. | - |
| 17 | 41 | + | - | - |
| 19 | 15 | N.T. | N.T. | + |
| 21 | 77 | N.T. | N.T. | + |
| 22 | 38 | + | - | + |
| 27 | 18 | + | - | + |
| 250 | N.T. | ± | N.T. | N.T. |

Remarks:
1) Above table shows a case of a nurse infected with hepatitis by needle prick accident. Time indicates number of week(s) elapsed after the accident. The patient, the source of the blood collected, was also tested positive for GORa antibody.
2) Result of the tests for Chiron's HCV are shown in * column for comparison.
3) N.T. means "not tested".

Table 5: Comparison of EIA using spGOR2 antibody and Western blot analysis using fusion protein

| No. | EIA | West. | No. | EIA | West. | No. | EIA | West. | No. | EIA | West. |
|---|---|---|---|---|---|---|---|---|---|---|---|
| 1 | 28 | - | 26 | ≥2000 | + | 51 | 1603 | + | 76 | 53 | - |
| 2 | 57 | - | 27 | 71 | - | 52 | 53 | - | 77 | ≥2000 | + |
| 3 | 107 | - | 28 | 63 | - | 53 | 55 | - | 78 | ≥2000 | + |
| 4 | ≥2000 | + | 29 | 1557 | + | 54 | 1554 | + | 79 | ≥2000 | + |
| 5 | 474 | - | 30 | 37 | - | 55 | 52 | ± | 80 | ≥2000 | + |
| 6 | 50 | - | 31 | 34 | - | 56 | 1512 | + | 81 | 386 | ± |
| 7 | 134 | - | 32 | 87 | - | 57 | 910 | - | 82 | ≥2000 | + |
| 8 | ≥2000 | + | 33 | ≥2000 | + | 58 | ≥2000 | + | 83 | 1554 | + |
| 9 | 1662 | - | 34 | 102 | - | 59 | 35 | - | 84 | 896 | ± |
| 10 | 51 | - | 35 | 63 | - | 60 | 110 | ± | 85 | ≥2000 | + |
| 11 | 807 | ± | 36 | 61 | - | 61 | 29 | - | 86 | 49 | - |
| 12 | 62 | - | 37 | 32 | - | 62 | 1941 | + | 87 | 75 | - |
| 13 | 101 | - | 38 | 1039 | + | 63 | 55 | - | 88 | 1630 | + |
| 14 | 99 | - | 39 | 30 | - | 64 | 789 | + | 89 | 33 | - |
| 15 | 1886 | + | 40 | 1731 | + | 65 | ≥2000 | + | 90 | 1283 | + |
| 16 | ≥2000 | + | 41 | 49 | - | 66 | 1945 | + | 91 | ≥2000 | + |
| 17 | 77 | - | 42 | 265 | ± | 67 | 53 | - | 92 | 35 | - |
| 18 | ≥2000 | + | 43 | ≥2000 | + | 68 | 200 | - | 93 | 64 | - |
| 19 | 397 | ± | 44 | 51 | - | 69 | 39 | - | 94 | ≥2000 | + |
| 20 | 80 | - | 45 | 98 | - | 70 | 119 | - | 95 | 101 | ± |
| 21 | ≥2000 | - | 46 | 34 | - | 71 | 167 | - | 96 | ≥2000 | + |
| 22 | ≥2000 | + | 47 | ≥2000 | + | 72 | 1582 | + | 97 | 69 | ± |
| 23 | ≥2000 | + | 48 | ≥2000 | + | 73 | 469 | - | 98 | 729 | ± |
| 24 | ≥2000 | + | 49 | 87 | - | 74 | ≥2000 | + | 99 | ≥2000 | + |
| 25 | 68 | - | 50 | ≥2000 | + | 75 | 607 | + | 100 | 807 | ± |

Remarks: "West." indicates Western blot analysis

## Sequence List

Sequence list 1. GOR gab DNA

GAATTCCCCC TGCCCTCTGA TCAGCCTGCC CAGAGCTTCG GGCTCCGGGT 50
GCCCCAGATG CACAACCAGG CCTCCGCATT TGTGGACATC CAGGCGGAGC 100
CCCAGAACAG GGGTCCGGCG GTGCCCCCAG CGTGTCTCAA GATGGTGACG 150
GAGGCGTCCT ACTTCCCTGC GCAGAGGGGA TCGGCCTGCT GCTTGCCAGC 200
CGCCCCAAGG CTGACAGAGA GGCCCTCGGG AGTCCGCATC TCAGCCCCCA 250
GGAAGAGGAA GACGATCGCC CAATCTTCCA GCCCTTGCTT GGTCACAGGT 300
TGCACAGATG CCAAGAGAAC CCGGGTGGCC AGCAGCAGCC AACGCTCCAG 350
TGGCTCCAAG GTCGGCAGAC AGCCAGGGAA GACGCGCAAC AGGTCAGGGA 400
TGGCATGCAA GACCACCACC ACCATCAGCT CTAAGCGAAT CGTCCGTCGT 450
CCATCCCTAC CGAGTTTGAA GAAACCTATT ATCCTAAGAA GGTCTGGGTG 500
CCAAGTCCCC ACCGTCCTCC GCCGAGGCTA TCTCCAACTG TTCACCGAAG 550
AGTGTCTCAA GTTCTGCGCC TCCAAGCAGG AGGCCGTGGA GAAGGCGCTG 600
AACGAGGAGA AGGTGGCCTA CGACTGCAGC CCCAACAAGA ACAGGTACCT 650
GAACGTGGTC CTGAACACCC TCAAGAGACT GAAGGGCCTG ACCCCCAGCT 700
GCCGCCCTGT ACAGCCGCCT CCATGCCGGG CCTCAGCAGG CCAGGAGTTC 750
CTGCTCAGCC AGGACCAGCT CAAGGAGAAC GGCTACCCCT TCCCGCACCC 800
CGAGCGGCCC GGAGGCGCCG TCCTCTTCAC TGGCCAGGGG AAGGGGCCCG 850
GCGACTCCTC CTGCAGGGTC TGCTGCCGTT GTGGCACCGA GTACCTGGTG 900
TCCTCCTCGG GCCGCTGTGT ACGCGACCAG TTGTGTTATT ATCACTGGGG 950
GCGGGTCCGC TCGAGCCAGG TGGCTGGAGG CCGGGTTAGC CAGTACACCT 1000
GCTGTGCAGC TGCTCCTGGC TCTGTGGGCT GCCAGGTGGC AAAGCAGCAC 1050
GTGCGGGACG GCCGCAAGGA CAGCCTCGAT GGCTTCGTGG AGACCTTCAA 1100
GAAAGAGTTG TCCAGAGACG CTTATCCAGG AATCTACGCC TTGGACTGTG 1150
AGATGTGCTA CACCACGCAT GGCCTAGAGC TGACCCGCGT CACCGTGGTG 1200
GACGCCGACA TGCGAGTGGT GTACGACACC TTCGTCAAGC CCGACAACGA 1250
GATCGTGGAC TACAACACCA GGTTTTCCGG AGTCACCGAG GCCGACGTCG 1300
CCAAGACGAG CATCACCTTG CCCCAAGTGC AAGCCATCCT GCTGAGCTTT 1350
TTCAGCGCCC AAACCATCCT CATCGGGCAC AGCCTGGAGA GCGATCTGCT 1400
GGCCCTGAAG CTCATCCACA GCACCGTGCT GGACACGGCC GTGCTCTTCC 1450
CGCACTACCT GGGTTTCCCC TACAAGCGTT CCCTCAGGAA TCTCGCGGCC 1500
GACTACCTGG GACAGATCAT CCAGGACAGC CAGGACGGCC ACAAGTGGAG 1550
CGAGGACGCA AACGCCTGCC TGCAGCTGGT GATGTGGAAG GTCCGACAGC 1600
GCGCCCAGAT CCAGCCACGC CACCGGTCCG CCTCTCCCGC CGCCCTGGCC 1650
TGTCCTTGGC CCCAGGCCCC TTCCACAACC GCCATCAGTC CCGAGAGCTC 1700
ACCCTGTCCA CCTCGCCGCA AGGCCAAAGA AACCGGAGCA GTCGACGGCA 1750
GGAGAGGGCA AAAAGCCAAG AGTAACCCCA ACCGGCCACT CCCAGTCCCC 1800
CGGAATCCCT GCCGCGGACC CTCGGGCCTG TCCCCATCCC TCTGCCCTTC 1850
CCAGACCTCT GTCCTTCCAC TAATCGCCTC CCGCAGCACC GAGCCACCAC 1900
TCCCAGTCCC CCGAGTCCCT GCCGCGCCCC CTCGCGCCTG TCCACATCCC 1950
TCTGCCCATC CGAGACCTCT GTCCTTACAC CACTAGCCAC CCCACGTGGG 2000
ACTTCCATGG CCTCTGAGTA CAAGGCCAGC CCCCCGGCCC ACCAGCTTTC 2050
TGAATGTGTG CTTACCTGTT 2070

Sequence list 2. GOR gab protein

EFPLPSHQPA QSFGLRVPQM HNQASAFVDI QAEPQNRGPA VPPACLKMVT 50
EASYFPRQRG SACCLPAAPR LTERPSGVRI SAPRKRKTIA QSSSPCLVTG 100
CTDAKRTRVA SSSQRSSGSK VGRQPGKTRN RSGMACKTTT TISSKRIVRR 150
PSLPSLKKPI ILRRSGCQVP TVLRRGYLQL FTEECLKFCA SKQEAVEKAL 200
NEEKVAYDCS PNKNRYLNVV LNTLKRLKGL TPSSMPGLSR AALYSRLQEF 250
LLSQDQLKEN GYPFPHPERP GGAVLFTGQG KGPGDSSCRV CCRCGTEYLV 300
SSSGRCVRDQ LCYYHWGRVR SSQVAGGRVS QYTCCAAAPG SVDCQVAKQH 350
VRDGRKDSLD GFVETFKKEL SRDAYPGIYA LDCEMCYTTH GLELTRVTVV 400
DADMRVVYDT FVKPDNEIVD YNTRFSGVTE ADVAKTSITL PQVQAILLSF 450
FSAQTILIGH SLESDLLALK LIHSTVLDTA VLFPHYLGFP YKRSLRNLAA 500

DYLGQIIQDS QDGHNSSEDA NACLQLVMWK VRQRAQIQPR HRSASPAALA 550
CPWPQAPSTT AISPESSPCP PRRKAKETGA VDGRRGQKAK SNPNRPLPVP 600
RNPCRGPSGL SPSLCPSQTS VLPLIASRST EPPLPVPRVP AAPPRACPHP 650
SAHPRPLSLH H 661

Sequence list 3. GOR47-1 RNA

CCUGUCCACC UCGCCGCAAG GCCAAAGAAA CCGGAGCAGU CGACGGCAGG 50
AGAGGGCAAA AAGCCAAGAG UAACCCCAAC CGGCCACUCC CAGUCCCCCG 100
GAAUCCCUGC CGCGGACCCU CGGGCCUGUC CCCAUCCCUC UGCCCUUCCG 150
AGACCUCUGU CCUUCC 166

Sequence list 4. GOR47-1 RNAC

GGAAGGACAG AGGUCUGGGA AGGGCAGAGG GAUGGGGACA GGCCCGAGGG 50
UCCGCGGCAG GGAUUCCGGG GGACUGGGAG UGGCCGGUUG GGGUUACUCU 100
UGGCUUUUUG CCCUCUCCUG CCGUCGACUG CUCCGGUUUC UUUGGCCUUG 150
CGGCGAGGUG GACAGG 166

Sequence list 5. GOR47-1 DNA

CCTGTCCACC TCGCCGCAAG GCCAAAGAAA CCGGAGCAGT CGACGGCAGG 50
AGAGGGCAAA AAGCCAAGAG TAACCCCAAC CGGCCACTCC CAGTCCCCCG 100
GAATCCCTGC CGCGGACCCT CGGGCCTGTC CCCATCCCTC TGCCCTTCCG 150
AGACCTCTGT CCTTCC 166

Sequence list 6. GOR47-1 DNAC

GGAAGGACAG AGGTCTGGGA AGGGCAGAGG GATGGGGACA GGCCCGAGGG 50
TCCGCGGCAG GGATTCCGGG GGACTGGGAG TGGCCGGTTG GGGTTACTCT 100
TGGCTTTTTG CCCTCTCCTG CCGTCGACTG CTCCGGTTTC TTTGGCCTTG 150
CGGCGAGGTG GACAGG 166

Sequence list 7. GOR47-1 protein

CPPRRKAKET GAVDGRRGQK AKSNPNRPLP VPRNPCRGPS GLSPSLCPSQ 50
TSVLP 55

Sequence list 8. spGOR1

CPPRRKAKET GAVDGRRGQK AKSNPNRPL 29

Sequence list 9. spGOR2

GRRGQKAKSN PNRPLPVPRN PCRGPSG 27

Sequence list 10. spGORa-epi

GRRGQKAKSN PNRPL 15

Sequence list 11. spGORb-epi

VAKQHVRDGR KDSLDGFV 18

## Claims

1. An oligopeptide spGORb-epi comprising the following amino acid sequence:
   V A K Q H V R D G R K D S L D G F V (Y) or analogs thereof.

2. The oligopeptide according to claim 1, wherein said amino acid sequence comprises V A K Q H V R D G R K D S L D G F V.

3. A non-A, non-B hepatitis antibody detection reagent comprising the amino acid sequence according to claim 1 as antigen.

4. A non-A, non-B hepatitis related antibody detection reagent comprising a fusion protein of the amino acid sequence according to claim 1 and a conventional protein for its expression.

5. A non-A, non-B hepatitis specific monoclonal or polyclonal antibody reactive with an antigen comprising the amino acid sequence according to claim 1.

6. A non-A, non-B hepatitis related antigen detection reagent comprising the specific antibodies according to claim 5.

# Fig. 1

```
λgt11 β-galactosidase ORF
----------------- CTGATGGAAACCAGCCATCGCCATCTACTGCACACGGAAGAAGAAGGCA
-----------------  L  M  E  T  S  H  R  H  L  L  H  T  E  E  E  G

CATGGCTGAATATCGACGGTTTCCATATGGGGATTGGTGGCGACGACTCCTGGAGCCCGT
T  W  L  N  I  D  G  F  H  M  G  I  G  G  D  D  S  W  S  P

                     → GOR47-1 ORF
CAGTATCGGCGGAATTCCCCTGTCCACCTCGCCGCAAGGCCAAAGAAACCGGAGCAGTCG
S  V  S  A  E  F  P  C  P  P  R  R  K  A  K  E  T  G  A  V

ACGGCAGGAGAGGGCAAAAAGCCAAGAGTAACCCCAACCGGCCACTCCCAGTCCCCCGGA
D  G  R  R  G  Q  K  A  K  S  N  P  N  R  P  L  P  V  P  R

ATCCCTGCCGCGGACCCTCGGGCCTGTCCCCATCCCTATGCCCTTCCCAGACCTCTGTCC
N  P  C  R  G  P  S  G  L  S  P  S  L  C  P  S  Q  T  S  V

 ←
TTCCGGAATTCCAGCTGAGCGCCGGTCGCTACCATTACCAGTTGGTCTGGTGTCAAAAAT
L  P  E  F  Q  L  S  A  G  R  Y  H  Y  Q  L  V  W  C  Q  K

AATAATAACCGGGCAGGCCATGTCTGCCCGTATTTCGCGTAAGGAAATCC
stop codons
```

Fig. 2

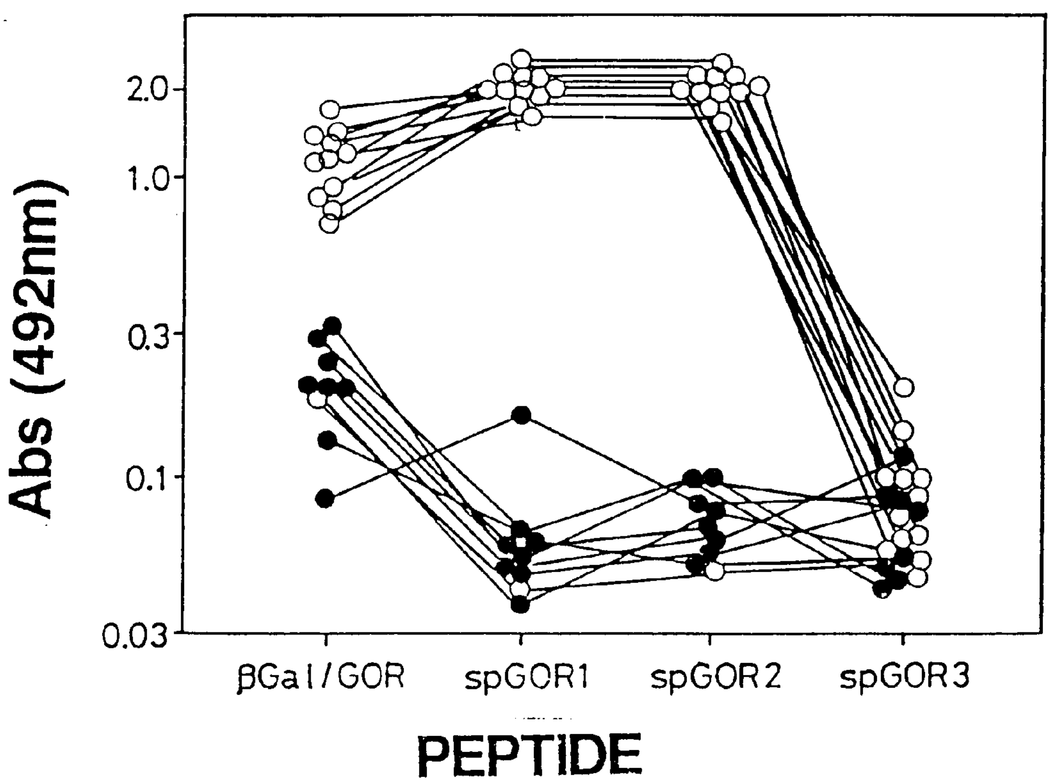
Abs (492nm)
2.0
1.0
0.3
0.1
0.03
βGal/GOR
spGOR1
spGOR2
spGOR3
PEPTIDE

# Fig. 3

GOR47-1

```
   primer G1
CCTGTCCACC TCGCCGCAAG GCCAAAGAAA CCGGAGCAGT CGACGGCAGG AGAGGGCAAA
GGACAGGTGG AGCGGCGTTC CGGTTTCTTT GGCCTCGTCA GCTGCCGTCC TCTCCCGTTT

AAGCCAAGAC TAACCCCAAC CGGCCACTCC CAGTCCCCCG GAATCCCTGC CGCGGACCCT
TTCGGTTCTC ATTGGGGTTG GCCGGTGAGG GTCAGGGGGC CTTAGGGACG GCGCCTGGGA

CGGGCCTGTC CCCATCCCTC TGCCCTTCCC AGACCTCTGT CCTTCC
GCCCGGACAG GGGTAGGGAG ACGGGAAGGG TCTGGAGACA GGAAGG
                               primer G2
```

Fig. 4

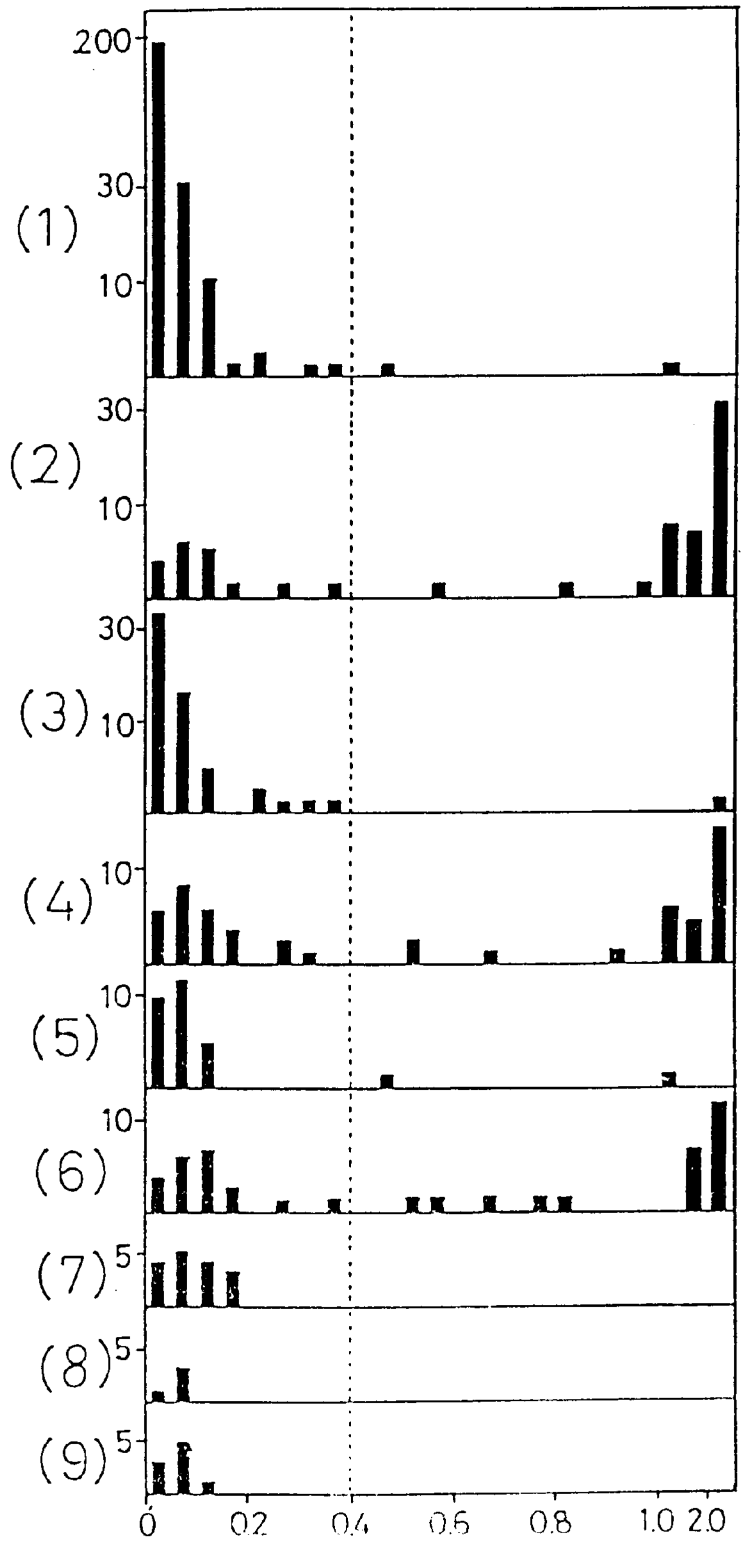


Abs (492nm)

Fig. 5

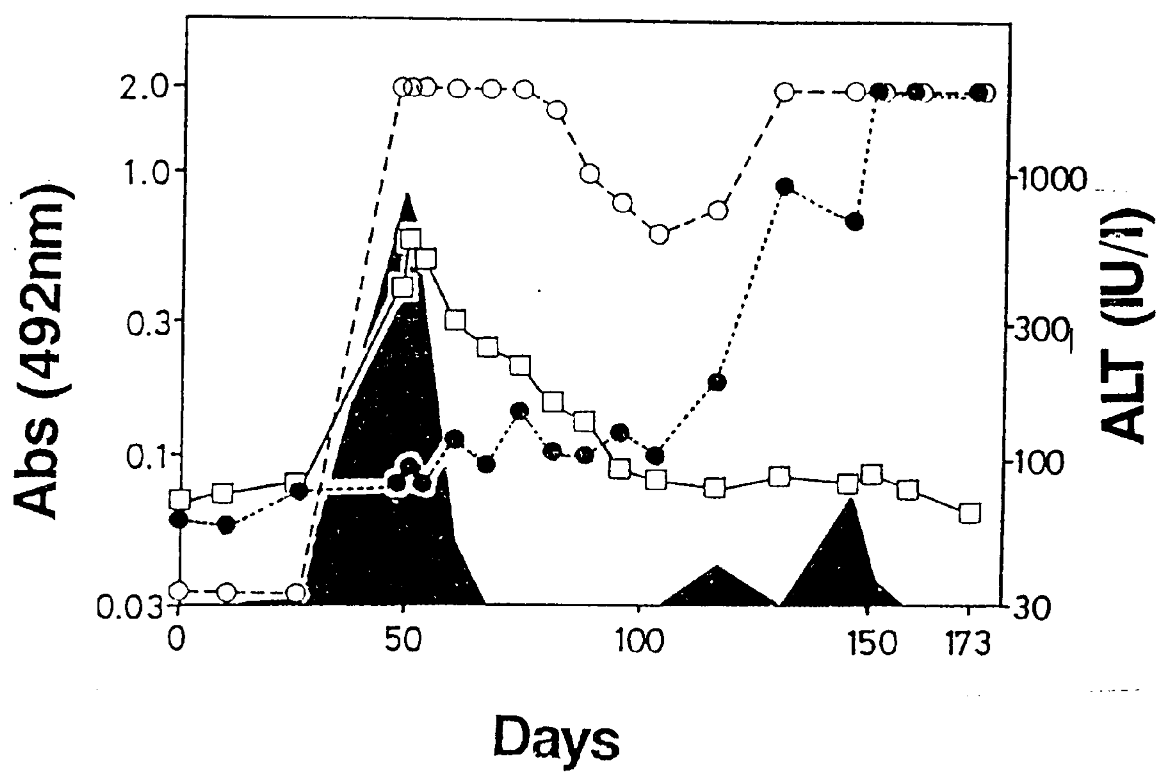
Abs (492nm)
2.0
1.0
0.3
0.1
0.03
ALT (IU/l)
1000
300
100
30
0
50
100
150
173
Days

Fig. 6

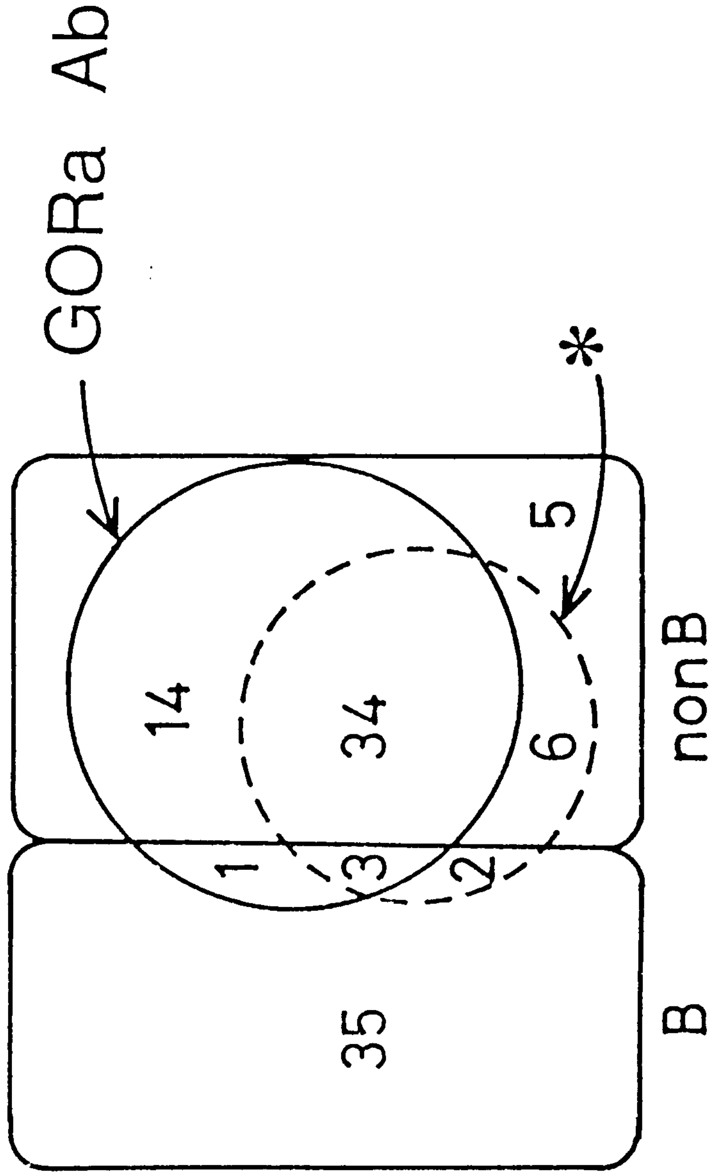

# Fig. 7

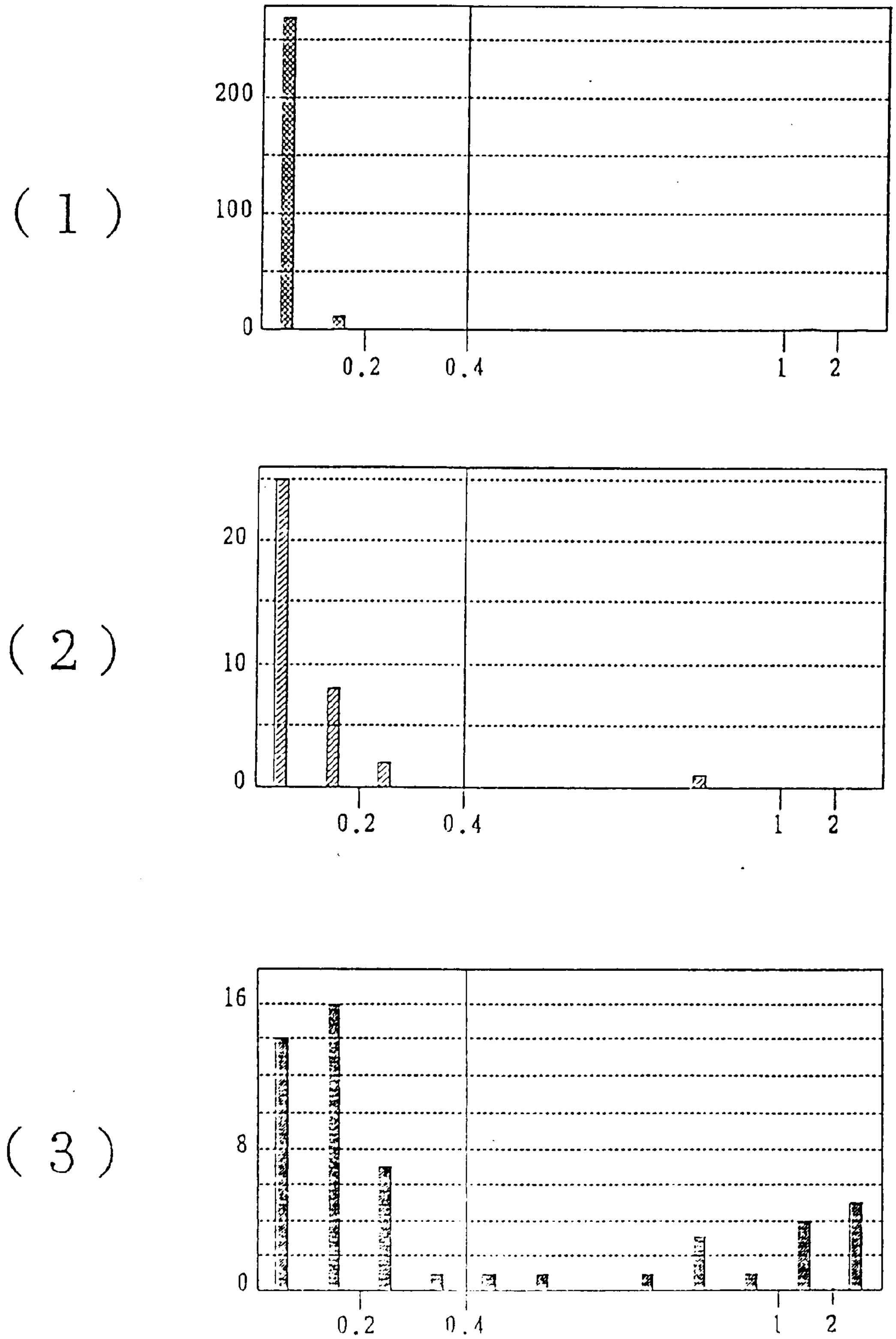

Fig. 8

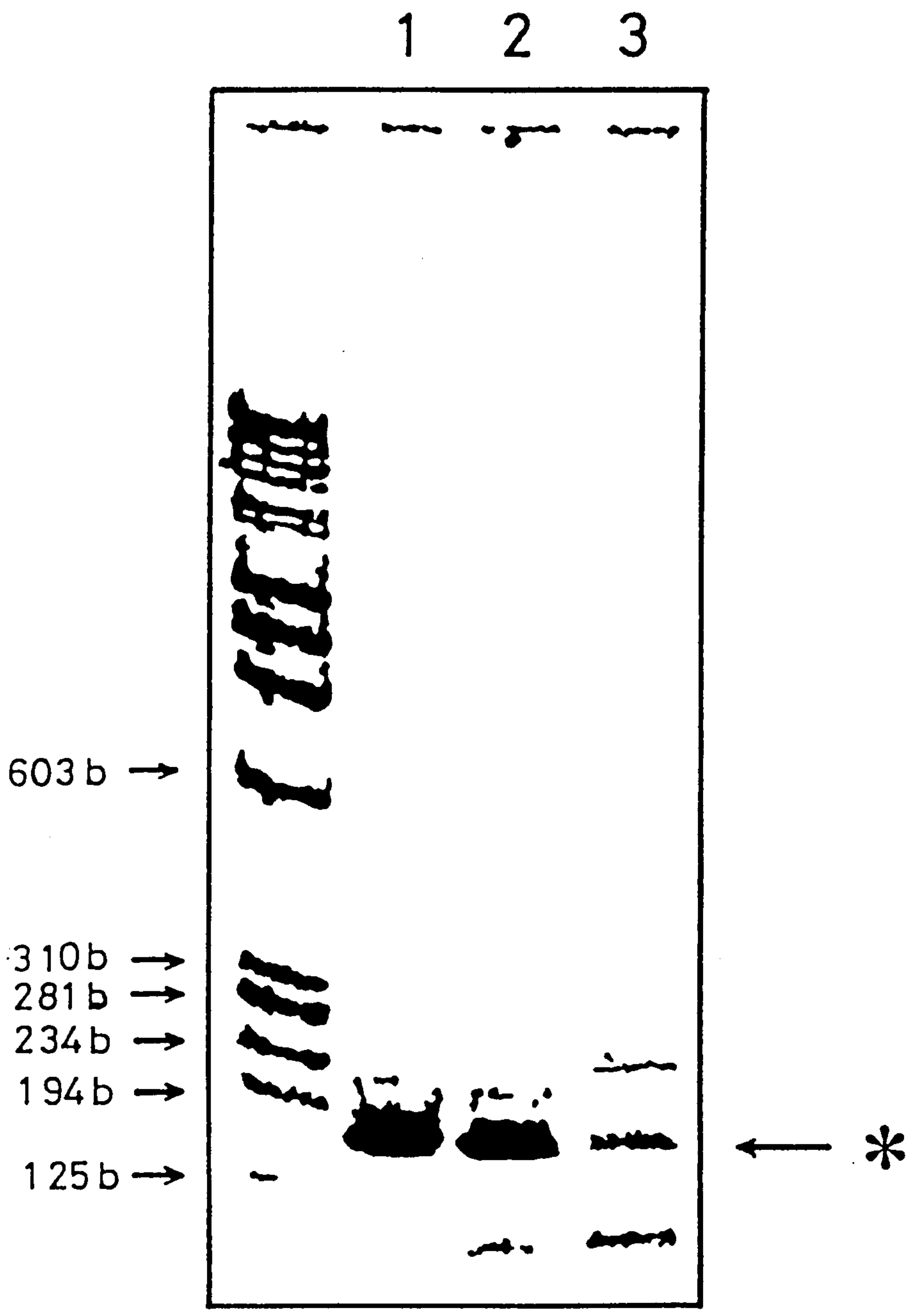

European Patent Office

# EUROPEAN SEARCH REPORT

Application Number

EP 92 30 1009

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | LANCET THE. vol. 336, no. 8728, 8 December 1990, LONDON GB pages 1400 - 1403; S. MISHIRO ET AL.: 'NON-A, NON-B HEPATITIS SPECIFIC ANTIBODIES DIRECTED AT HOST-DERIVED EPITOPE: IMPLICATION FOR AN AUTOIMMUNE PROCESS.' * the whole document * | 1-6 | C07K7/08<br>G01N33/576<br>G01N33/577<br>C12P21/08<br>//C12N15/51 |
| | ----- | | |

TECHNICAL FIELDS SEARCHED (Int. Cl.5 )

C07K
G01N
C12N
C12P

The present search report has been drawn up for all claims

| Place of search | Date of completion of the search | Examiner |
|---|---|---|
| THE HAGUE | 20 MAY 1992 | RYCKEBOSCH A.O. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)